# EUROPEAN PATENT APPLICATION

(11) **EP 2 094 064 A1**
(43) Date of publication of application: **26.08.2009**
(21) Application number: 07849916.7
(22) Date of filing: 29.11.2007
(51) Int. Cl.: H05B 37/02, A61M 21/02, A61N 5/06

(54) **LIGHT SOURCE, LIGHT SOURCE SYSTEM AND ILLUMINATION DEVICE**

(30) Priority: 08.12.2006 JP 2006331947
(71) Applicant: Sharp Kabushiki Kaisha, Osaka-shi, Osaka 545-8522 (JP)
(72) Inventor: UCHIUMI, Tadashi, Osaka-shi, Osaka 545-8522 (JP); KANEKO, Takashi, Osaka-shi, Osaka 545-8522 (JP); TSUBOI, Masanori, Osaka-shi, Osaka 545-8522 (JP); YAMANAKA, Atsushi, Osaka-shi, Osaka 545-8522 (JP)
(74) Representative: Müller - Hoffmann & Partner
(86) International application number: PCT/JP2007/073085
(87) International publication number: WO 2008/069101

(57) **Abstract**

A light source and an illumination device are provided for making it possible to adjust influence of light over a living body while keeping good brightness and color taste of illumination light. A conversion characteristic in the degree of effect indicative of a relationship in a light wavelength between a living body reaction characteristic and an isochromatic characteristic of the visual perception is derived out and the light emitting intensity of a plurality of light emitters with different wavelength characteristics is controlled in accordance with the conversion characteristic, so as to adjust influence of light over a living body while keeping brightness and color taste of illumination light.

## Description

### TECHNICAL FIELD

The present invention relates to a light source that is used mainly for illumination and that can control a level of influence on a living body, and to a light source system and an illumination device to which the light source is applied.

### BACKGROUND OF THE INVENTION

From the ancient time, the human race has generally been living under sunlight as natural light in their course of evolution. It is considered, therefore, that the human physiological mechanisms corresponding to the cycle of light and darkness, such as day and night, have been developed. Among such mechanisms, the rhythm of sleep and awakening is a reaction triggered in principle by light, and is equivalent to the rhythm of 24-hour cycle (circadian rhythm). This biological rhythm actually has a cycle slightly longer than the 24-hour cycle, but being exposed to light in the morning advances the phase of the biological rhythm cycle to reset the biological rhythm, in which way the biological rhythm is adjusted to the day/night cycle of environmental light.

In general, a hormone called melatonin is secreted from the pineal body of the brain at night in a time zone between a pre-hypnagogic time and the first half of sleep. Melatonin secretion has an effect of causing a body temperature to drop and facilitating falling asleep. It is clearly known, however, that being exposed to relatively intensive light in the nighttime inhibits the secretion of melatonin to affect sleep after the light exposure, while, on the contrary, being exposed to relatively intensive light in the daytime raises an amount of melatonin secretion in the nighttime.

In this manner, light is deeply concerned with biological rhythm adjustment, melatonin secretion inhibition, etc., so that a quantitative and qualitative change of light including a time-dependent change of light, such as light/darkness cycle, has a great influence on a physiological mechanism, especially on the biological rhythm.

In the modern society, time of living in the indoor environment is raising as the trend of nocturnal lifestyle is in progress due to the development of illuminating equipment, which has lead to the light environment with less change in light and darkness in day and night. Because of this, the biological rhythm is not adjusted well to the 24-hour cycle, which brings about such a social problem of damage to people' s physical and mental health, such as sleep disturbance and insomnia. Under such a social situation, a control technique for quantitatively and qualitatively controlling illuminating light in the living space becomes more important.

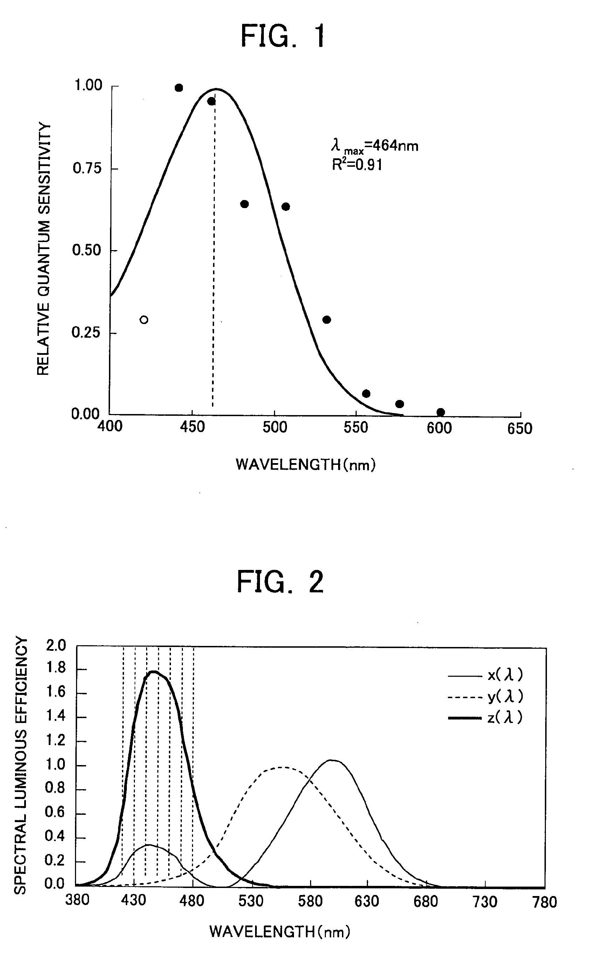

In connection with this, a paper on the relation between light wavelength and melatonin secretion influencing the biological rhythm, such as Nonpatent Literature 1, has been published. This Nonpatent Literature 1 explains how the wavelength of light to which a person is exposed in the nighttime influences an amount of melatonin to be secreted by using the form of an action spectrum. Nonpatent Literature 1 reports that a sensitivity characteristic for wavelength-influenced melatonin secretion inhibition results in a sensitivity characteristic shown in Fig. 1.

According to the above characteristic, if a plurality of light sources having different energy quantities in a wavelength range around 464 nm at which a sensitivity peak is formed are prepared and are changed for each other in light emission, inhibition and noninhibition of melatonin secretion can be changed for each other. A technique adopting this approach is, for example, disclosed in Patent Document 1, which describes an illuminating method and illumination device that carry out melatonin secretion inhibition and biological rhythm adjustment by changing a light source having a large quantity of energy in a wavelength range of 410 nm to 505 nm at which a sensitivity for melatonin secretion inhibition is high and a light source not having a large quantity of energy in that wavelength range for each other.
Patent Document 1: Japanese Laid-Open Patent Publication No. 2005-310654
Nonpatent Literature 1: George C. Brainard, et al. : "Action Spectrum for Melatonin Regulation in Humans: Evidence for a Novel Circadian Photoreceptor", The Journal of Neuroscience, August 15, 2001, 21(16), pp. 6405-6412.

### DISCLOSURE OF THE INVENTION

### PROBLEMS TO BE SOLVED BY THE INVENTION

In the above wavelength range of 410 nm to 505 nm, however, a melatonin secretion inhibition effect, that is, a level of influence on a living body (this influence is also called "biological effect" or "bioeffect" in this specification) is high, and an influence on the brightness and color change of illuminating light is also great. In other words, simply raising or lowering light in the above wavelength range causes brightness and the way of look of an object color to change in connection with a change in a level of influence on the living body. The above technique, therefore, is not necessarily a satisfactory technique for application to an illumination for daily usage.

It is thought that the human is capable of sensing the brightness of light and the color of an object because the human eyes are equipped with a sensor corresponding to three primary colors of light. Fig. 2 depicts spectral sensitivities corresponding to the human eyes. These spectral sensitivities are referred to as color matching functions, which are represented as functions x(λ), y(λ), and z(λ) that have large sensitivities in wavelength ranges of red, green, and blue, respectively. Now, let attention be paid to the curve of the function z(λ). This function z(λ) has a sensitivity peak at a wavelength of blue light of about 440 nm to 450 nm, but has a sensitivity less than half of the peak sensitivity for similar blue-color-based light of a wavelength close to, for example, 420 nm and 480 nm in sensitivity comparison with the wavelength that gives the sensitivity peak. From a reversed point of view, this means that if light of a wavelength of 420 nm is to give the human eyes a sensation as strong as a sensation that light of a wavelength of 450 nm would give, light of the wavelength of 420 nm requires light intensity approximately two times its original intensity. Changing the intensities of light of the wavelength of 450 nm and light of the wavelength of 420 nm, therefore, have influences on the way of feeling brightness or color that are greatly different, so that dealing with light having different wavelengths on the same basis is not appropriate even if the light is within the same wavelength range of 410 nm to 505 nm as shown in Patent Document 1.

A level of influence on a living body by illuminating light varies depending on individual variations, physical conditions, etc. Because of this, such control of separately using light having a strong bioinfluence level and light not having strong bioinfluence level for different purposes as described in Patent Document 1 may bring about a problem of an insufficient or an excessive effect because of a difference in the physical nature or physical condition of a illumination user.

The present invention was conceived in view of the above problems, and it is therefore the object of the present invention to provide a light source and illumination device that are capable of controlling a bioinfluence while well maintaining brightness and hue that are important as properties of illuminating light.

### MEANS FOR SOLVING THE PROBLEMS

As described above, the human eyes' sensitivity varies depending on wavelengths. Selecting a wavelength range to be used while considering the variation of the human eyes' sensitivity, therefore, enables controlling a bioinfluence by illumination while well maintaining brightness and hue sensed by a person.

In order to solve the above problem, according to the present invention, a converted effect level characteristic α(λ) is introduced, which represents the relation between a bioeffect characteristic by a light wavelength and the color matching characteristic of the eyesight. By carrying out intensity control based on this characteristic α(λ), a bioinfluence level can be changed as the brightness and hue of light is maintained.

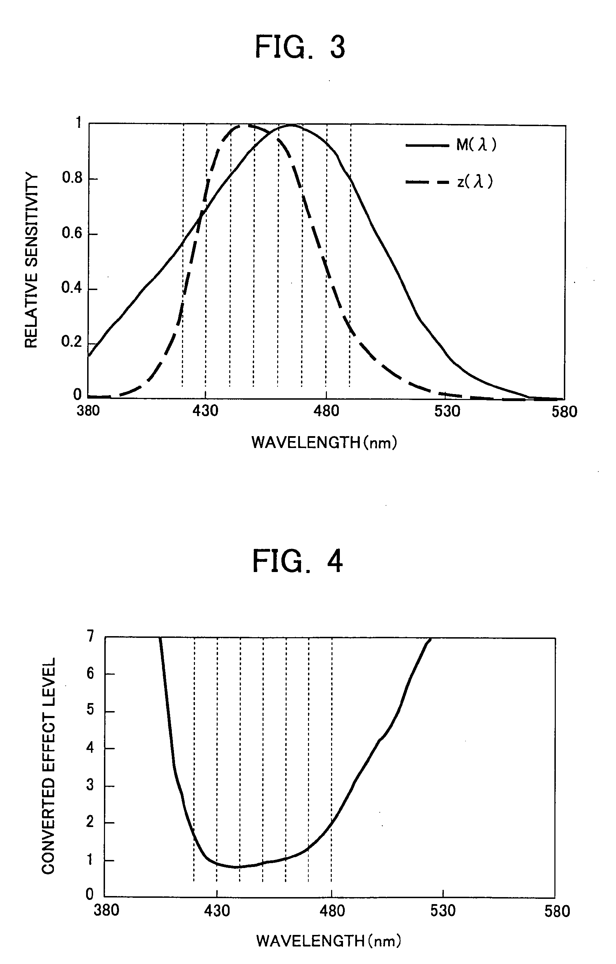

The converted effect level characteristic α(λ) is derived as α(λ)=M(λ)/z(λ) for each wavelength, from the action spectrum M(λ) for melatonin secretion inhibition of Fig. 1 and the color matching function z(λ) on blue light of Fig. 2. The derived characteristic α(λ) means the size ratio between an influence level contributing to melatonin secretion inhibition and an influence level contributing to a visual sense of color for each light wavelength. Specifically, a wavelength with a larger α(λ) value has a higher level of influence on melatonin secretion inhibition, while a wavelength with a smaller α(λ) value has a higher level of influence on a color sense. The value of α(λ) is calculated after normalizing the action spectrum M(λ) and the color matching function z(λ) to set a peak value of both curves to 1, as shown in Fig. 3. Fig. 4 depicts the converted effect level characteristic α(λ). This characteristic α(λ) demonstrates that a wavelength having the highest level of influence on a color sense is present near a wavelength range of 430 nm to 440 nm, and that a level of influence on melatonin secretion inhibition raises as a wavelength range goes away from that wavelength to the short wavelength side and to the long wavelength side.

Changing the spectrum of light while considering the above characteristic α(λ) provides a light source and illumination device that are capable of controlling a degree of melatonin secretion inhibition, i.e., a bioinfluence level while suppressing a change in the brightness or hue of light as much as possible.

A first configuration of a light source according to the present invention having the above features provides a light source comprising at least two different types of emitters A and B having wavelength characteristics different from each other, wherein the emitters A and B have bioeffect properties that influence an awakening level or a hormone secretion of a living body through exposure to light from the emitters A and B, and wherein the emitter A offers a higher bioeffect level through light exposure at the same light emission intensity in comparison with the emitter B, while the emitter B offers a lower bioeffect level through light exposure at the same light emission intensity in comparison with the emitter A.

The emitters A and B offer a weaker bioeffect through light exposure when a light emission intensity of the emitter A is raised while lowering the light emission intensity of the emitter B, whereas the emitters A and B offer a stronger bioeffect through light exposure when the light emission intensity of the emitter A is lowered while raising the light emission intensity of the emitter B.

A bioeffect level through light exposure is varied by lowering a light emission intensity of the emitter B when the light emission intensity of the emitter A is raised but by raising the light emission intensity of the emitter B when the light emission intensity of the emitter A is lowered.

Light emission intensities of the emitters A and B are controlled so that a blue color component of a chromaticity of synthesized light generated by both emitters becomes substantially constant or is a value within a given range, based on a converted effect level calculated from an action spectrum characteristic on a bioeffect by light and a color matching function on blue light.

More specifically, the emitter A has a light emission energy peak in a wavelength range of about 420 nm to 440 nm, and the emitter B has a light emission energy peak in a wavelength range of about 440 nm to 510 nm.

Alternatively, the emitter A has a light emission energy peak in a wavelength range of about 440 nm to 470 nm, and the emitter B has a light emission energy peak in a wavelength range of about 470 nm to 510 nm.

A second configuration of the light source according to the present invention provides a light source capable of changing a peak value of a light emission wavelength within a given wavelength range, wherein the light source has bioeffect properties that influence an awakening level or hormone secretion of a living body through exposure to light from the light source, the light source varying the bioeffect by changing a peak value of a light emission wavelength.

More specifically, the given wavelength range is at least in a range of 420 nm to 440 nm and in a range of 440 nm to 510 nm, the given wavelength range being switchable between both the ranges.

Alternatively, the given wavelength range is at least in a range of 440 nm to 470 nm and in a range of 470 nm to 510 nm, the given wavelength range being switchable between both the ranges.

For example, a vertical cavity surface emitting laser is used as the above light source capable of changing a peak value of a light emission wavelength.
Provided as another invention is a light source system that has bioeffect properties and that is capable of emitting light different in color from light emitted by the above light source in use of the light source described as the first and second configurations.

A first configuration of the light source system is a light source system that includes any one of the above light sources and an emitter having wavelength characteristics different from the wavelength characteristics of the light source.

A second configuration of the light source system is a light source system that includes any one of the above light sources and at least one emitter used together with the light source to produce white light.
An illumination device using any one of the above light source systems is provided as still another invention.
The illumination device using any one of the above light source systems controls the light emission intensity of each of light sources or emitters included in the light source system to vary a bioeffect level while keeping a general color rendering index of produced illuminating light equal to or more than a given value.

### EFFECTS OF THE INVENTION

The present invention having the above configurations offers the following effects. A light source of the present invention is capable of adjusting a level of influence on a living body caused by exposure to light emitted from the light source while keeping brightness and hue sensed through the light substantially the same brightness and hue.

A light source system including the above light source is capable of keeping the brightness and hue of light different in color from light from a single unit of the above light source substantially the same brightness and hue while adjusting a level of influence on a living body in the same manner as described above.

An illumination device configured to be able to acquire white light using the above light source system is capable of adjusting a level of influence on a living body caused by exposure to illuminating light while keeping not only the brightness and hue, i.e., color temperature, but color rendering properties of the illuminating light at a given level.

### BRIEF DESCRIPTION OF DRAWINGS

[Fig. 1] Fig. 1 is a graph of the wavelength characteristics (action spectrum) of light on the human sensitivity for melatonin secretion inhibition;
[Fig. 2] Fig. 2 is a diagram of spectral sensitivities (color matching functions) corresponding to the human eyes;
[Fig. 3] Fig. 3 is a diagram of the relation between a color matching function z(λ) and a sensitivity characteristic M(λ) for melatonin secretion inhibition;
[Fig. 4] Fig. 4 is a diagram of the wavelength characteristics of a converted effect level α(λ) represented by the ratio between the sensitivity characteristic for melatonin secretion inhibition and the color matching function;
[Fig. 5] Fig. 5 is a diagram of a configuration example of a light source and a light source system according to a first embodiment of the present invention;
[Fig. 6] Fig. 6 is a diagram of examples of spectral distributions of illuminating light by the light source system of the first embodiment of the present invention;
[Fig. 7] Fig. 7 is a diagram of examples of spectral distributions of illuminating light by a light source system of a second embodiment of the present invention;
[Fig. 8] Fig. 8 is a diagram of a configuration example of a light source and a light source system according to a third embodiment of the present invention; and
[Fig. 9] Fig. 9 is a diagram of a configuration example of an illumination device according to a fourth embodiment of the present invention.

### EXPLANATION OF REFERENCE NUMERALS

100... light source system
101 to 104... emitter
110... light source
200... light source system
201, 202...light source
203, 204... emitter
300... illumination device
301... light source system
302... light source control portion
303... light characteristic analyzing portion
304... condition setting portion

### PREFERRED EMBODIMENTS OF THE INVENTION

Embodiments of a light source, a light source system, and an illumination device of the present invention will now be described in detail with reference to the drawings.

### FIRST EMBODIMENT

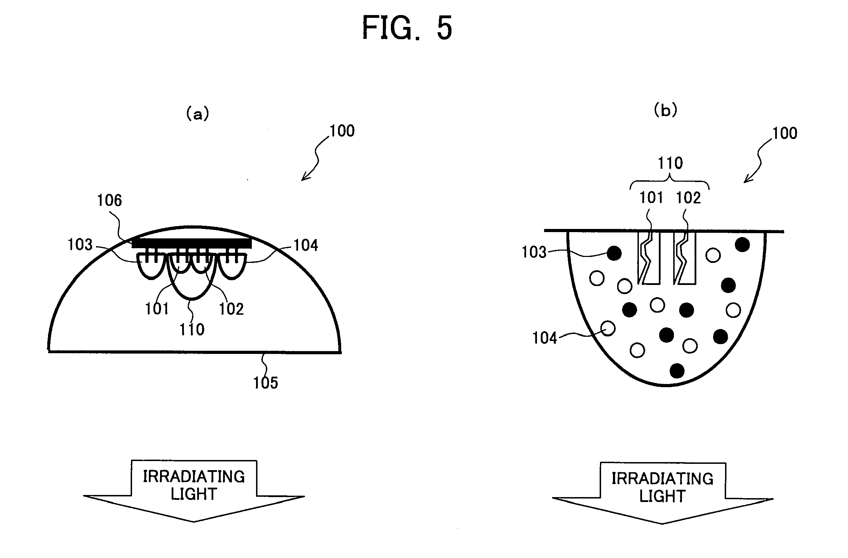

Fig. 5 depicts a configuration example of a light source and a light source system according to a first embodiment of the present invention. A light source system 100 is configured to include a light source 110 composed of a first emitter 101 and a second emitter 102, and third and fourth emitters 103 and 104. A control portion, which is not shown, is connected to the light source 110, and controls the light emission intensity of each of the emitters 101 and 102.

The emitter 101 and the emitter 102 have wavelength characteristics different from each other, and the emitter 101 has a light emission energy peak at a wavelength of about 430 nm while the emitter 102 has a light emission energy peak at a wavelength of about 490 nm. The emitters 103 and 104 have peak wavelengths of 540 nm and 646 nm, respectively. These emitters are configured in a specific combination of wavelengths so that simultaneous light emission by four emitters produces white light. The light source system 100 may be provided with a light diffuser plate 105 and a reflector plate 106 so that illuminating light produced by mixing together beams of light from four emitters is a result of efficient use of light from the emitters and that the illuminating light is emitted uniformly.

Both emitter 101 and emitter 102 among four emitters have light emission peaks at wavelengths that make an effect on melatonin secretion, i.e., bioeffect level high, where the values of M(λ) on the emitter 101 and on the emitter 102 are about 0.7 and 0.8, respectively, as shown in Fig. 3. The light emission intensities of these emitters are controlled to be able to significantly change a bioeffect level.

The emitter 101 and the emitter 102, however, show a great difference on a color matching function, which reveals that both emitters differ greatly in sensitivity to the human eyes. Specifically, having the light emission peak wavelength of 430 nm, the emitter 101 shows a high relative sensitivity of about 0.8 when the peak of z(λ) is set to 1. The emitter 102, on the other hand, has its light emission peak wavelength of 490 nm, thus showing a low relative sensitivity of around 0.25 on z(λ). When a bioeffect level is adjusted by changing the light emission intensities of both emitters, therefore, simply paying attention to a level of influence on the bioeffect of each emitter to change the light emission intensity may result in a wide change in the brightness and color of light before and after bioeffect level adjustment. In this embodiment, to keep the brightness and color of light substantially constant before and after bioeffect level adjustment, the light emission intensity of each of the emitters is controlled by considering the bioinfluence level of each emitter and a relative sensitivity ratio represented by the color matching function z(λ) of each emitter.

A solution to an optimization problem is used as an algorithm for determining the light emission intensities of the above four emitters. The Newton's method is employed as a solution applicable to a problem pattern involving a plurality of unknown values to calculate the light emission intensity of each emitter that makes a bioeffect level the maximum and the minimum under a specific condition. The result of calculation is shown in a table 1. Conditions to be set in finding a solution include chromaticity coordinate values x=0.31±0.005 and y=0.32±0.005 in the XYZ color specification system, a general color rendering index of 80.0 (based on reference of CIE standard light D65), and constant radiant intensity. These conditions mean that the color of illuminating light is within a range of so-called white light, that accuracy in the way of look of an object color is kept at high level, and the luminance, i.e., brightness of illuminating light is maintained.

**[Table 1]**

| Bioeffect level | Emitter 1 (430 nm) | Emitter 2 (490 nm) | Emitter 3 (540 nm) | Emitter 4 (646 nm) |
|---|---|---|---|---|
| Maximum (113.6%) | 296.6 | 636.1 | 96.5 | 102.6 |
| Minimum (97.9%) | 341.7 | 381.3 | 113.1 | 97.3 |

The bioeffect level values on the table 1 represent percentages that are determined relative to the bioeffect level of standard light D65 that is set to 1. Numerical values representing the light emission intensities of the emitters are relative values, thus do not accompany specific unit. Control of each emitter in execution of bioeffect level adjustment tends to be carried out in such a way that the light intensity level of the first emitter is lowered while that of the second emitter is raised when the bioeffect level is raised, and, inversely, that the light intensity level of the first emitter is raised while that of the second emitter is lowered when the bioeffect level is lowered. The table 1 exhibits an example of the light emission intensities of the emitters that correspond to the maximum and the minimum of a bioeffect level acquired by the light source system of this embodiment. If an index value for the bioeffect level is additionally specified within the range between the maximum and the minimum as a condition to be set in finding a solution by the above procedure, the light emission intensity of each of the emitters that corresponds to the specified bioeffect level can be calculated.

In this manner, use of the light source system of the present invention enables changing a bioeffect level within a range of about 98% to 114% of the bioeffect level of standard light while keeping the brightness, chromaticity, or color temperature and color rendering properties of illuminating light substantially constant.

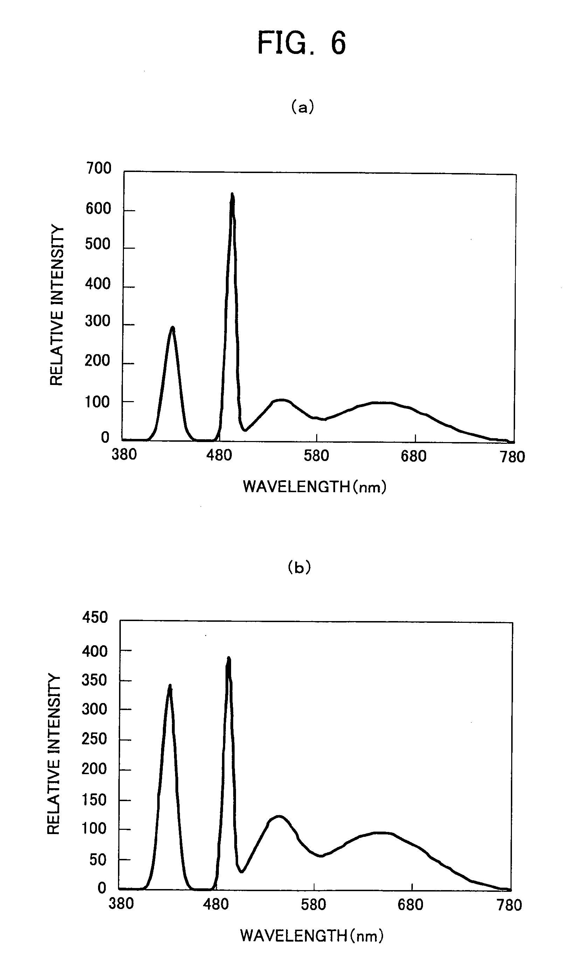

Fig. 6 depicts examples of spectral distributions of illuminating light that are acquired in the light emission intensity configuration shown in the table 1. Fig. 6(a) is an example of a spectral distribution that results when the bioeffect level is the maximum, and Fig. 6(b) is an example of a spectral distribution that results when the bioeffect is the minimum.

A latitude of adjustment of the above bioeffect level can be controlled by easing a condition to be given in the course of calculating the bioeffect level. For example, if the condition on the chromaticity coordinate value (x, y) is allowed to be wider range of 0.30≤x≤0.32 and 0.30≤y≤0.35, a range of an adjustable bioeffect level can be widened to a range of about 86% to 119% that is wider than the result of the table 1. Further easing the condition on the chromaticity coordinate value allows bioeffect level adjustment in a wider range. The same adjustment applies to the general color rendering index. If a value of the general color rendering index is allowed to be lower than 80.0, a bioeffect adjustment range can be expanded further. Such adjustment is carried out properly in accordance with the use and object of this light source system.

In this embodiment, an LED (Light-Emitting Diode) is used as the emitter to achieve a configuration in which the light emission intensity of each of the above four emitters can be controlled independently. Obviously, various types of emitters, such as a semiconductor laser and an EL element, may be used in replacement of the LED if the emitter has properties of having a peak at a specific wavelength.

A fluorescent material may be used as the third and fourth emitters in replacement of such a natural light emitter as LED. In such a case, the fluorescent material to be used receives light from the first and second emitters to emit light (excitation light emission), and has the same wavelength characteristics as the above emitter. Fig. 5(b) depicts a configuration example of the light source system 100 in a case of using the fluorescent material.

When the fluorescent material is used, controlling the light emission intensity independent of the fluorescent material's exciting light source is basically impossible. Setting the amount and density of the fluorescent material to a proper level so that desired illuminating light results, however, enables adjustment of a bioeffect level without significantly changing conditions of chromaticity, color rendering properties, etc., as shown in a table 2, although a variable range of the bioeffect level and the maintenance of color rendering properties become slightly inferior.

**[Table 2]**

| Third/fourth emitter | Bioeffect level | Chromaticity coordinate value (x) | Chromaticity coordinate value (y) | General color rendering index |
|---|---|---|---|---|
| Natural light | Maximum (113.6%) | 0.305 | 0.324 | 80.0 |
| | Minimum (97.9%) | 0.310 | 0.324 | 80.0 |
| Fluorescent material | Maximum (113.1%) | 0.302 | 0.306 | 82.1 |
| | Minimum (102.1%) | 0.313 | 0.314 | 77.7 |

### SECOND EMBODIMENT

A light source and a light source system according to a second embodiment of the present invention will then be described. A basic configuration of the light source system of the second embodiment is the same as that of the first embodiment, thus includes four emitters as the example of Fig. 5. Peak wavelengths of the first and second emitters of the second embodiment, however, are different from those of the first embodiment.

In the second embodiment, the light emission energy peak of the emitter 101 is at a wavelength of about 455 nm, while the light emission energy peak of the emitter 102 is at a wavelength of about 490 nm. The emitters 103 and 104, on the other hand, have peak wavelengths of 540 nm and 646 nm, respectively, as the emitters 103 and 104 of the first embodiment.

Comparison with the first embodiment reveals a feature that the peak wavelength of the first emitter 101 offers higher sensitivities on both melatonin secretion inhibition and visual sense of color, which can be observed in Fig. 3.

In this light source system, to adjust the bioinfluence level of illuminating light, a range of controlling the light emission intensities of four emitters is calculated within a range satisfying a given condition in the same manner as in the first embodiment. The result of calculation is shown in a table 3.

**[Table 3]**

| Bioeffect level | Emitter 1 (455 nm) | Emitter 2 (490 nm) | Emitter 3 (540 nm) | Emitter 4 (646 nm) |
|---|---|---|---|---|
| Maximum (107.7%) | 212.4 | 437.6 | 99.5 | 105.4 |
| Minimum (77.7%) | 243.6 | 0 | 129.1 | 94.4 |

As shown in the table 3, comparison with the light source system of the first embodiment reveals that a bioeffect level adjustable range expands in the direction of lowering the bioeffect level. The tendency of control of each emitter in execution of bioeffect level adjustment is such that the light intensity level of the first emitter is lowered while that of the second emitter is raised when the bioeffect level is raised, and, inversely, that the light intensity level of the first emitter is raised while that of the second emitter is lowered when the bioeffect level is lowered.

In this manner, use of the light source system of the second embodiment enables changing a bioeffect level within a range of about 78% to 108% of the bioeffect level of standard light while keeping the brightness, chromaticity, or color temperature and color rendering properties of illuminating light substantially constant.

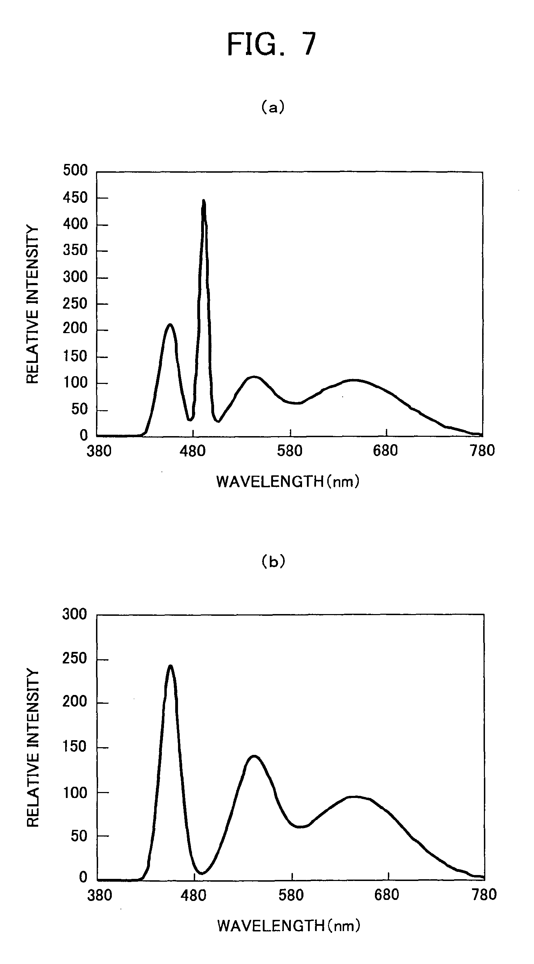

Fig. 7 depicts examples of spectral distributions of illuminating light that are acquired in the light emission intensity configuration shown in the table 3. Fig. 7(a) is an example of a spectral distribution that results when the bioeffect level is the maximum, and Fig. 7(b) is an example of a spectral distribution that results when the bioeffect is the minimum.

A latitude of adjustment of the above bioeffect level can be controlled by easing a condition on the chromaticity, etc. , of light as desired illuminating light in the same manner as in the first embodiment.

In this embodiment, in the same manner as in the first embodiment, an LED (Light-Emitting Diode) is used as the emitter to achieve a configuration in which the light emission intensity of each the above four emitters can be controlled independently. Obviously, various types of emitters, such as a semiconductor laser and an EL element, may be used in replacement of the LED if the emitter has properties of having a peak at a specific wavelength.

A fluorescent material may be used as the third and fourth emitters in replacement of such a natural light emitter as LED. In such a case, the fluorescent material to be used receives light from the first and second emitters to emit light (excitation light emission), and has the same wavelength characteristics as the above emitter.

In both first and second embodiments, more than four types of emitters may be used to make up the light source system. In such a case, the number of peak wavelengths raises, which eases a condition on necessary wavelength characteristics. As a result, a bioeffect level adjustment range can be widened when chromaticity and general color rendering index conditions remain the same, compared to the above embodiment in which four types of emitters are used, and a color rendering index of a higher value can be achieved when the bioeffect level adjustment range remains at the same level, compared to the case of four types of emitters.

In both first and second embodiments, a single unit of the light source composed of the first and second emitters may solely be used. In such a case, according to the configuration of the light source of the above embodiment, illuminating light comes to have a main component of a blue color component, which makes the light source unfit for an illumination that illuminates the entire indoor space. By carrying out control based on the same idea as described above, however, such a light source can be used as an illumination device capable of adjusting a bioeffect level while keeping the color of illuminating light substantially constant.

### THIRD EMBODIMENT

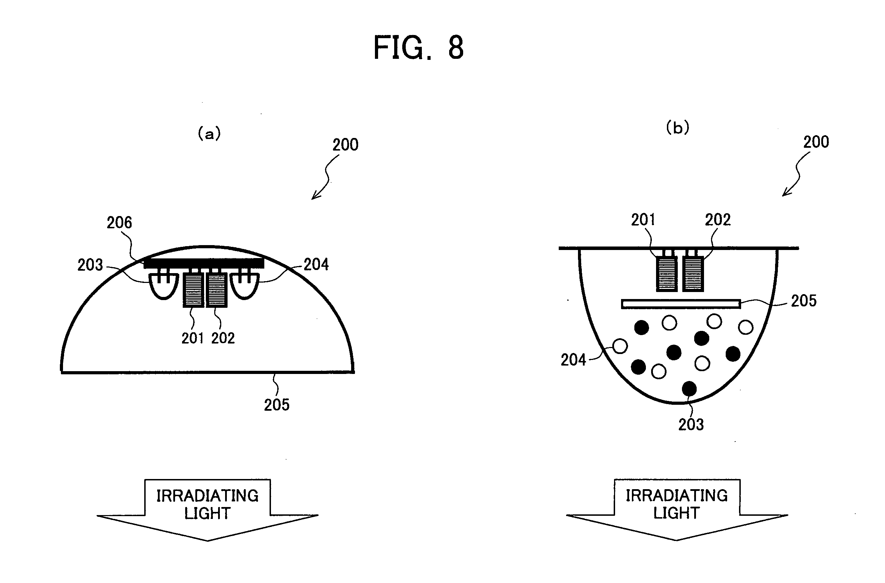

A light source and a light source system according to a third embodiment of the present invention will then be described. Fig. 8 depicts a configuration example of the light source and the light source system of this embodiment.

The light source system 200 is configured to include the first light source 201, a second light source 202, and emitters 203 and 204. A control portion, which is not shown, is connected to the light sources 201 and 202, and controls the light emission intensity of each of the light sources 201 and 202.

Each of the above two light sources is a light source capable of changing a peak value of a light emission wavelength within a given wavelength range, and is configured using, for example, a wavelength variable vertical cavity surface emitting laser disclosed in Published Japanese Translation No. 2004-529501 of the PCT International Publication. This wavelength variable vertical cavity surface emitting laser includes a light-generating layer that emits light when supplied with current, and a phase control element that modulates a light wavelength by a position-dependent electrooptical effect. The phase control element is configured as two stages of elements having directions of wavelength modulation different from each other, so that the surface emitting laser serves as a light source capable of causing a wavelength to shift toward the longer wavelength side and toward the shorter wavelength side as well.

These light sources are configured to be capable of changing a wavelength in at least part of a wavelength range of 420 nm to 440 nm and in at least part of a wavelength range of 440 nm to 510 nm, thus used as the light sources 201 and 202.

The emitter 203 and the emitter 204 have light emission peak wavelengths of 540 nm and 646 nm, respectively, and are configured in a combination of wavelength characteristics so that simultaneous light emission from the emitters 203 and 204 together with the above two emitters produces white light. The emitters 203 and 204 may be replaced with such a natural light emitter as LED, or with a fluorescent material that receives light from the light sources 201 and 202 to emit light having the above wavelength characteristics. Fig. 8(b) depicts a configuration example of the light source system 200 in a case of using the fluorescent material. In this case, the light source system 200 may be provided with a light diffuser plate 205 so that beams of light from two light sources 201 and 202 are used efficiently, and that illuminating light finally produced by mixing together the beams of light is emitted uniformly.

Both light sources 201 and 202 have light emission peaks at wavelengths that make an effect on melatonin secretion, i.e., bioeffect level high, as the emitters 101 and 102 of the first and second embodiments. The light emission intensities of these light sources are controlled to be able to significantly change a bioeffect level. These light sources, however, differ greatly from each other in color sensitivity to the human eyes, so that the light emission intensity of each of the light sources is controlled by considering a color sensitivity ratio of the light source in the same manner as in the first and second embodiments.

Compared to the light source system of the first and second embodiments, the light source system of this embodiment is more advantageous in that a bioeffect level adjustment range can be widened through control of only one type of a light source because the light sources 201 and 202 can be controlled in illuminating light wavelength. The light source system of this embodiment is capable of offering wavelength characteristics that cannot be realized by the LED now in use, thus widening a bioeffect level adjustment range and allowing construction of a light source system that offers a higher general color rendering index.

The light sources 201 and 202 using the above wavelength variable vertical cavity surface emitting laser may be configured in such a way that the wavelength variable ranges of the light sources 201 and 202 may include at least part of a wavelength range of 440 nm to 470 nm and at least part of a wavelength range of 470 nm to 510 nm, in addition to the wavelength variable ranges as described above. In this case, a range of adjustment of a bioeffect level by illuminating light can be changed as in the case of the first and second embodiments. Such a change is made properly in accordance with the use and object of this light source system.

### FOURTH EMBODIMENT

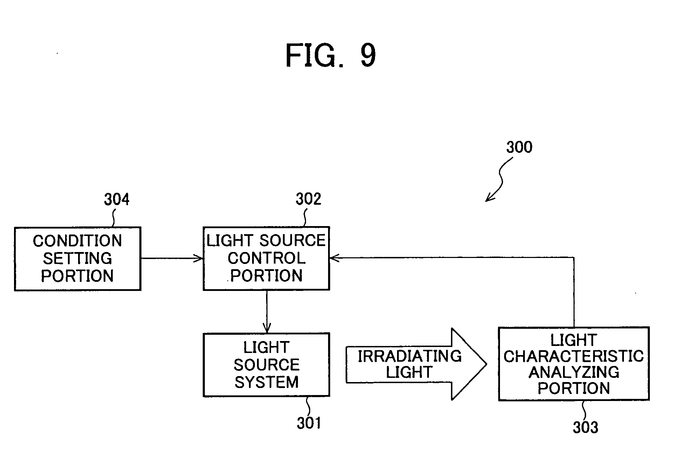

An illumination device according to a fourth embodiment of the present invention will then be described. Fig. 9 depicts a configuration example of the illumination device of this embodiment. The illumination device 300 is configured to include a light source system 301, a light source control portion 302, a light characteristic analyzing portion 303, and a condition setting portion 304.

The light source system 301 includes a light source similar to the light source of the first to third embodiments that has bioeffect properties that influence the awakening level or hormone secretion of a living body exposed to illuminating light from the light source, and also includes an emitter other than the light source that may contain a fluorescent material so that illuminating light generated by the light source system is so-called white light.

The light source control portion 302 controls the intensity of each light source or emitter included in the light source system in accordance with a desirable bioeffect level expected to be given by illuminating light from the light source system. When intensity control is carried out, the intensity balance of each light source or emitter is determined using the solution to an optimization problem as described in the first embodiment. When the intensity of each light source or emitter is changed, the characteristics of each light source and emitter are stored in advance in a memory means not shown, and a voltage or current to be given to the light source and emitter is changed in accordance with the characteristics. The actual characteristics of each light source and emitter, however, may change depending on service environments, secular change, etc., so that feedback control may be carried out whenever necessary in accordance with the result of analysis of illuminating light. A condition given to the optimization problem is imparted from the condition setting portion 304, which will be described later.

The light characteristic analyzing portion 303 is configured to include a light-receiving portion and a means that analyzes the characteristics of received light. The light characteristic analyzing portion 303 thus receives illuminating light from the light source system to analyze the intensity, wave length characteristics, etc., of received light. The result of analysis is imparted to the light source control portion 302. The imparted analysis result at least contains information indicative of light energy quantity, such as radiant flux in each given wavelength interval included in illuminating light, and may further contain the position of illuminating light on the chromaticity coordinate (vector value) and a general color rendering index value based on a reference of standard light (D65, etc.), which index corresponds to the color temperature of illuminating light.

The condition setting portion 304 regulates characteristic parameters of illuminating light by the light source system, and may be configured to include an operation portion operated by a user. The characteristic parameters of illuminating light to be regulated include the luminance of light or illuminance of light at a given position, the color temperature or chromaticity of light, a general color rendering index, and an index value for a bioeffect level. These characteristic parameters may be stored in advance in a memory means not shown, or may be specified by the user through the operation portion. Stored or specified characteristic parameters are imparted to the light source control portion 302, which works out a solution to the optimization problem with a condition given as the imparted characteristic parameters to determine the balance of each light source or emitter.

The above characteristic parameters are determined based on the following way of thinking. For example, when the user wants to work intensively on jobs and reading or to wake up with a clear-headed feeling in the morning, a characteristic parameter creating a high bioeffect level is specified or selected for the purpose of raising an awakening level. When the user wants to spend relaxed time or to get into a physical condition for a sound sleep, in contrast, a characteristic parameter creating a low bioeffect level is specified or selected. Specification or selection of a characteristic parameter may be carried out by the user through the operation portion, or may be carried out in such a way that a preset parameter is automatically called up in response to the arrival of a specified time.

Because the light source system 301 uses emitted light as illuminating light, the light source system 301 may be configured to have a diffuser plate that is disposed around or in front of the light source and the emitter to ensure uniform illumination with emitted light, or may be configured to have a lens that focuses emitted light to use it as spotlight.

## Claims

1. A light source comprising at least two different types of emitters A and B having wavelength characteristics different from each other, wherein
the emitters A and B have properties of biological effects that influence an awakening level or a hormone secretion of a living body through exposure to light from the emitters A and B, and wherein
the emitter A offers a higher biological effect level through light exposure at the same light emission intensity in comparison with the emitter B, while the emitter B offers a lower biological effect level through light exposure at the same light emission intensity in comparison with the emitter A.

2. The light source as defined in claim 1, wherein
the emitters A and B offer a weaker biological effect through light exposure when a light emission intensity of the emitter A is raised while lowering the light emission intensity of the emitter B, whereas the emitters A and B offer a stronger biological effect through light exposure when the light emission intensity of the emitter A is lowered while raising the light emission intensity of the emitter B.

3. The light source as defined in claim 1 or 2, wherein
a biological effect level through light exposure is varied by lowering a light emission intensity of the emitter B when the light emission intensity of the emitter A is raised but by raising the light emission intensity of the emitter B when the light emission intensity of the emitter A is lowered.

4. The light source as defined in any one of claims 1 to 3, wherein
a biological effect level through light exposure is varied by controlling light emission intensities of the emitters A and B so that a blue color component of a chromaticity of synthesized light generated by both emitters becomes substantially constant, based on a converted effect level calculated from an action spectrum characteristic on a biological effect by light and a color matching function on blue light.

5. The light source as defined in any one of claims 1 to 3, wherein
a biological effect level through light exposure is varied by controlling light emission intensities of the emitters A and B so that a blue color component of a chromaticity of synthesized light generated by both emitters is within a given range, based on a converted effect level calculated from an action spectrum characteristic on a biological effect by light and a color matching function on blue light.

6. The light source as defined in any one of claims 1 to 5, wherein
the emitter A has a light emission energy peak in a wavelength range of about 420 nm to 440 nm, and the emitter B has a light emission energy peak in a wavelength range of about 440 nm to 510 nm.

7. The light source as defined in any one of claims 1 to 5, wherein
the emitter A has a light emission energy peak in a wavelength range of about 440 nm to 470 nm, and the emitter B has a light emission energy peak in a wavelength range of about 470 nm to 510 nm.

8. A light source capable of changing a peak value of a light emission wavelength within a given wavelength range, wherein
the light source has properties of biological effect that influence an awakening level or hormone secretion of a living body through exposure to light from the light source, the light source varying the biological effect by changing a peak value of a light emission wavelength.

9. The light source as defined in claim 8, wherein
the given wavelength range is at least in a range of 420 nm to 440 nm and in a range of 440 nm to 510 nm, the given wavelength range being switchable between both the ranges.

10. The light source as defined in claim 8, wherein
the given wavelength range is at least in a range of 440 nm to 470 nm and in a range of 470 nm to 510 nm, the given wavelength range being switchable between both the ranges.

11. The light source as defined in any one of claims 8 to 10, wherein
the light source is a vertical cavity surface emitting laser.

12. A light source system comprising:
the light source of any one of claims 1 to 11; and
an emitter having wavelength characteristics different from wavelength characteristics of the light source.

13. A light source system comprising:
the light source of any one of claims 1 to 11; and
at least one emitter that is used with the light source to produce white light.

14. An illumination device using the light source system of claim 12 or 13, wherein
the illumination device controls a light emission intensity of each light source or emitter included in the light source system while keeping a general color rendering index of produced illuminating light equal to or more than a given value to change a biological effect level.
